# EUROPÄISCHE PATENTSCHRIFT

(11) **EP 3 388 104 B1**
(45) Veröffentlichungstag und Bekanntmachung des Hinweises auf die Patenterteilung: **20.11.2019**
(21) Anmeldenummer: 18165761.0
(22) Anmeldetag: 04.04.2018
(51) Int. Cl.: A61M 25/00

(54) **SCHLAUCHLEITUNG UND VERFAHREN ZU DEREN HERSTELLUNG**
FLEXIBLE HOSE AND METHOD FOR ITS MANUFACTURE
CONDUITE FLEXIBLE ET SON PROCÉDÉ DE FABRICATION

(30) Priorität: 11.04.2017 DE 102017206154
(43) Veröffentlichungstag der Anmeldung: 17.10.2018
(73) Patentinhaber: B. Braun Melsungen AG, 34212 Melsungen (DE)
(72) Erfinder: WEIß, André, 34302 Guxhagen (DE); KAHLEN, Oliver, 34281 Gudensberg (DE)
(74) Vertreter: Patentanwälte Ruff, Wilhelm, Beier, Dauster & Partner mbB

(56) Entgegenhaltungen:
- EP-A2- 0 263 645
- EP-A2- 1 905 476
- WO-A2-2008/052764
- DE-A1- 3 310 870
- DE-A1- 4 037 641
- US-A1- 2005 085 761
- US-A1- 2008 249 501

## Beschreibung

Die Erfindung betrifft eine Schlauchleitung mit
- einem zwischen einem ersten Schlauchende und einem zweiten Schlauchende axial erstreckten Schlauchmantel mit einer Mantelaußenfläche und einer Mantelinnenfläche,
- mindestens einem von der Mantelinnenfläche umschlossenen und zwischen den Schlauchenden axial erstreckten Axialdurchlass,
- mindestens einem im Wesentlichen radial durch den Schlauchmantel erstreckten Radialdurchlass, der den mindestens einen Axialdurchlass fluidleitend mit der Mantelaußenfläche verbindet und
- mindestens einem zwischen dem Radialdurchlass und dem zweiten Schlauchende angeordneten und den mindestens einen Axialdurchlass in Richtung des zweiten Schlauchendes fluiddicht verschließenden Verschluss.

Eine derartige Schlauchleitung ist im Bereich der Fluidtechnik allgemein bekannt und kann zum radialen Ausströmen eines Fluids verwendet werden. Zu diesem Zweck weist die bekannte Schlauchleitung einen Radialdurchlass, der seitlich von einem Axialdurchlass abzweigt, sowie einen Verschluss auf. Der Verschluss ist in axialer Richtung hinter dem Radialdurchlass in dem Axialdurchlass angeordnet und verschließt diesen fluiddicht. Der Radialdurchlass wird mittels Bohren oder Stanzen in den Schlauchmantel der Schlauchleitung eingebracht. Der Verschluss ist in Gestalt eines separaten Bauelements oder eines Füllmaterials in dem Axialdurchlass angeordnet.

Siehe, zum Beispiel, DE4037641 A1, US2005085761 A1, US2008249501 A1, DE3310870 A1 und WO2008052764 A2.

Aufgabe der Erfindung ist es, eine Schlauchleitung der eingangs genannten Art zu schaffen, die besonders vorteilhaft herstellbar ist und eine funktionsgerechte Abdichtung gewährleistet.

Diese Aufgabe wird dadurch gelöst, dass der mindestens eine Radialdurchlass durch einen im Wesentlichen radial nach innen gewölbten Schlauchmantelabschnitt gebildet ist, der abschnittsweise von dem übrigen Schlauchmantel entlang einer Trennlinie getrennt ist, wobei der mindestens eine Verschluss dadurch gebildet ist, dass der Schlauchmantelabschnitt an der Mantelinnenfläche angelegt und fluiddicht mit dieser verbunden ist. Durch die erfindungsgemäße Lösung ist es möglich, auf ein zusätzliches Bauelement zum Verschließen des Axialdurchlasses zu verzichten. Stattdessen wird der Verschluss erfindungsgemäß durch den abschnittsweise von dem übrigen Schlauchmantel entlang der Trennlinie getrennten, nach innen gewölbten, an der Mantelinnenfläche angelegten und mit dieser fluiddicht verbundenen Schlauchmantelabschnitt gebildet. Durch die abschnittsweise Trennung von dem übrigen Schlauchmantel und die nach innen gerichtete Wölbung des Schlauchmantelabschnitts wird hierbei gleichsam der Radialdurchlass gebildet. Demzufolge kann auf eine gesonderte Einbringung des Radialdurchlasses, beispielsweise mittels Stanzen oder dergleichen, verzichtet und somit Material eingespart werden. Unter einer abschnittsweisen Trennung im Sinne der Erfindung ist zu verstehen, dass der Schlauchmantelabschnitt jedenfalls entlang eines Randbereichs einstückig mit dem übrigen Schlauchmantel verbunden ist. Unter einem Radialdurchlass im Sinne der Erfindung ist eine Öffnung zu verstehen, durch die ein Fluid aus der Schlauchleitung herausfließen und/oder in die Schlauchleitung hineinfließen kann, wodurch ein Fluidstrom vom axialen Durchlass zum radialen Durchlass und/oder umgekehrt ermöglicht ist.

Die erfindungsgemäße Lösung eignet sich in besonders vorteilhafterweise für Katheterschläuche, insbesondere für mehrlumige Zentralvenenkatheter. Die Erfindungsgemäße Lösung kann aber auch im Bereich der allgemeinen Fluidtechnik, dort wo seitliche Austrittsöffnungen an Schlauchleitungen relevant sind, verwendet werden.

In Ausgestaltung der Erfindung - der Schlauchleitung gemäß dem unabhängigen Anspruch 1 sowie der davon abhängigen Unteransprüche - weist die Trennlinie in einer radial auf den Radialdurchlass gerichteter Blickrichtung einen zungenförmigen Verlauf auf. Der Verlauf der Trennlinie umfasst insoweit einen ersten axial verlaufenden Linienabschnitt, einen im Wesentlichen halbkreisförmigen Linienabschnitt und einen dritten im Wesentlichen antiparallel und zu dem ersten Linienabschnitt beabstandet verlaufenden Linienabschnitt. Durch diesen zungenförmigen Verlauf der Trennlinie kann eine besonders dichte Anlage und eine vorteilhafte Verbindung des Schlauchabschnitts mit der Mantelinnenfläche erreicht werden.

In weiterer Ausgestaltung der Erfindung ist die Schlauchleitung ein medizinscher Katheterschlauch, insbesondere ein Zentralvenenkatheter, das erste Schlauchende ein proximales Katheterschlauchende, das zweite Schlauchende ein distales Katheterschlauchende, der Axialdurchlass ein erstes Lumen und der Radialdurchlass ein Katheternebenkanal. Auf diese Weise wird ein besonders vorteilhaft herstellbarer und funktionsgerecht abgedichteter Katheterschlauch gebildet.

In weiterer Ausgestaltung der Erfindung ist zumindest ein zweites Lumen vorgesehen, das durch eine axial erstreckte Trennwand fluiddicht von dem ersten Lumen getrennt ist, wobei der Schlauchmantelabschnitt fluiddicht mit der Trennwand verbunden ist. Bei üblichen mehrlumigen Kathetern wird der Verschluss zwischen dem Katheternebenkanal und dem distalen Katheterschlauchende mittels Formschnur oder pastöser Formmassen gebildet. Dies ist aufwändig und kann technologiebedingt zu einer unvollständigen Abdichtung führen. Unter Umständen kann es deshalb zu einer Keimansiedlung und -vermehrung im Bereich des Verschlusses kommen. Infektionen können deshalb nicht ausgeschlossen werden. Diese Ausgestaltung der Erfindung behebt diese Nachteile und schafft einen besonders vorteilhaften mehrlumigen Katheter.

Die Erfindung betrifft zudem ein Verfahren zur Herstellung einer Schlauchleitung - gemäß dem unabhängigen Anspruch 5 sowie der davon abhängigen Unteransprüche - gemäß den vorstehenden Ausführungen.

Das erfindungsgemäße Verfahren umfasst die Schritte: Auftrennen des Schlauchmantels entlang der Trennlinie; Einwölben des derart erhaltenen Schlauchmantelabschnitts in radialer Richtung und Anlegen des Schlauchmantelabschnitts an der Mantelinnenfläche; fluiddichtes Verbinden des Schlauchmantelabschnitts mit der Mantelinnenfläche. Durch das erfindungsgemäße Verfahren kann der Verschluss ohne zusätzliche Bauelemente oder ein Verfüllen des Axialdurchlasses mit Formmasse geschaffen werden. Somit wird eine besonders kostengünstige Herstellung erreicht.

In weiterer Ausgestaltung der Erfindung erfolgt das Auftrennen mittels Laserschneidens. Durch diese Ausgestaltung des erfindungsgemäßen Verfahrens kann die Geometrie des Radialdurchlasses besonders maßhaltig und qualitätsgetreu hergestellt werden.

In weiterer Ausgestaltung der Erfindung erfolgt das fluiddichte Verbinden mittels Laserschweißens. Derart kann eine besonders zuverlässige Abdichtung erreicht werden. Sofern ebenfalls das Auftrennen des Schlauchmantels mittels Laser erfolgt, kann eine besonders investitionsarme Fertigung erreicht werden, da ein und dieselbe Technologie für die Herstellung der Radialöffnung und die Herstellung des Verschlusses Anwendung findet.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in einer schematischen Draufsicht eine gattungsgemäße Schlauchleitung nach dem Stand der Technik,
- Fig. 2: in einer teilweise geschnittenen Seitenansicht die gattungsgemäße Schlauchleitung nach Fig. 1,
- Fig. 3: in einer schematischen Draufsicht eine erste Ausführungsform einer erfindungsgemäßen Schlauchleitung,
- Fig. 4: in einer teilweise geschnittenen Seitenansicht die Schlauchleitung nach Fig. 3,
- Fig. 5: in einer teilweise geschnittenen Seitenansicht eine zweite Ausführungsform einer erfindungsgemäßen Schlauchleitung, die im Bereich der Medizintechnik vorgesehen ist, und
- Fig. 6: in einer schematischen Darstellung eine Ausführungsform des erfindungsgemäßen Verfahrens.

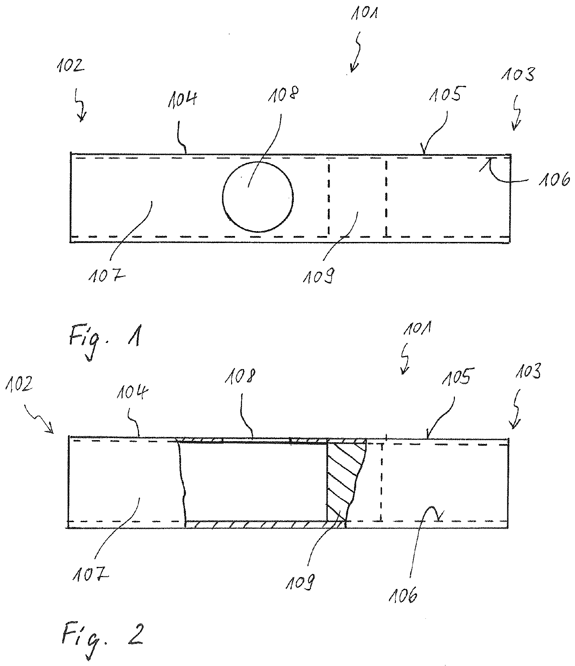

Eine gattungsgemäße Schlauchleitung 101 nach Fig. 1 und 2 ist im Bereich der Fluidtechnik vorgesehen. Die gattungsgemäße Schlauchleitung 101 weist einen zwischen einem ersten Schlauchende 102 und einem zweiten Schlauchende 103 axial erstreckten Schlauchmantel 104 mit einer Mantelaußenfläche 105 und einer Mantelinnenfläche 106 auf. Die gattungsgemäße Schlauchleitung 101 weist zudem einen Axialdurchlass 107 und einen Radialdurchlass 108 auf. Der Axialdurchlass 107 ist von der Mantelinnenfläche 106 umschlossen und erstreckt sich in axialer Richtung zwischen dem ersten Schlauchende 102 und dem zweiten Schlauchende 103. Der Radialdurchlass 108 erstreckt sich in Gestalt einer kreiszylindrischen Ausnehmung in radialer Richtung durch den Schlauchmantel 104 und bildet derart eine fluidleitende Verbindung von dem Axialdurchlass 107 zu der Mantelaußenfläche 105 der Schlauchleitung 101. Weiter weist die gattungsgemäße Schlauchleitung 102 einen Verschluss 109 auf. Der Verschluss 109 ist in axialer Richtung zwischen dem Radialdurchlass 108 und dem zweiten Schlauchende 103 angeordnet und verschließt derart den Axialdurchlass 107 einseitig in Richtung des zweiten Schlauchendes 103. Bei der gattungsgemäßen Schlauchleitung 101 ist der Verschluss 109 durch ein separates mit der Mantelinnenfläche 106 fluiddicht verbundenes Bauelement gebildet. Alternativ kann der Verschluss 109 durch eine plastische Füllmasse oder dergleichen gebildet sein. Der Radialdurchlass 108 ist bei der bekannten Schlauchleitung 109 durch ein vollständiges Heraustrennen eines Schlauchmantelabschnitts aus dem Schlauchmantel 104 mittels Stanzen, Schneiden oder dergleichen hergestellt.

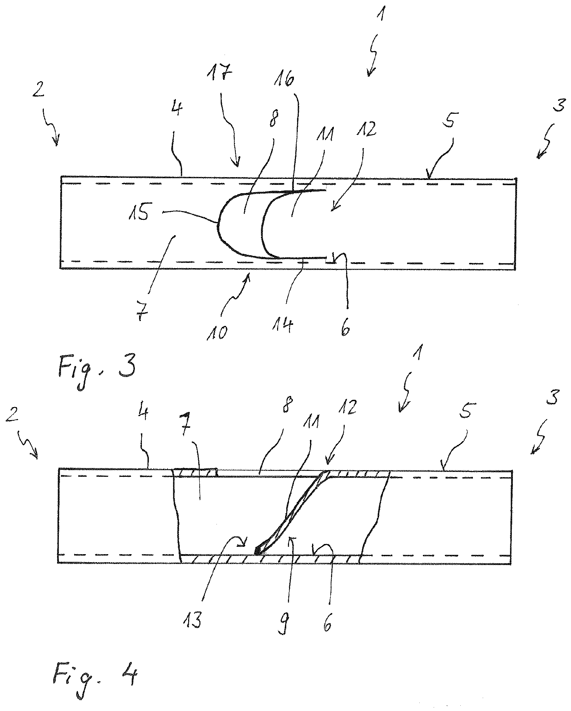

Eine erste Ausführungsform einer erfindungsgemäßen Schlauchleitung 1 nach den Fig. 3 und 4 ist im Bereich der Fluidtechnik zum radialen Ausströmen eines Fluids vorgesehen. Die erfindungsgemäße Schlauchleitung 1 weist einen zwischen einem ersten Schlauchende 2 und einem zweiten Schlauchende 3 axial erstreckten Schlauchmantel 4 mit einer Mantelaußenfläche 5 und einer Mantelinnenfläche 6 auf. Innerhalb des Schlauchmantels 4 und von der Mantelinnenfläche 6 umschlossen erstreckt sich zwischen den Schlauchenden 2, 3 ein Axialdurchlass 7. Von dem Axialdurchlass 7 im Wesentlichen in radialer Richtung abzweigend erstreckt sich ein Radialdurchlass 8 durch den Schlauchmantel 4, so dass eine fluidleitende Verbindung zwischen dem Axialdurchlass 7 und der Mantelaußenfläche 5 geschaffen ist. Der Radialdurchlass 8 ist durch Einwölben eines abschnittsweise von dem übrigen Schlauchmantel 4 entlang einer Trennlinie 10 getrennten Schlauchmantelabschnitts 11 gebildet. Der Schlauchmantelabschnitt 11 ist auf eine noch näher zu beschreibende Art und Weise entlang der Trennlinie von dem übrigen Schlauchmantel 4 gelöst und in einem Stirnendbereich 12 einstückig mit dem Schlauchmantel 4 verbunden. Wie anhand der teilweise geschnittenen Seitenansicht nach Fig. 4 ersichtlich ist, erstreckt sich der Schlauchmantelabschnitt 11 ausgehend von dem Stirnendbereich 12 radial schräg nach unten gerichtet bis zu einem dem Radialdurchlass 8 gegenüberliegenden Wandabschnitt 13 der Mantelinnenfläche 6 und liegt an dieser an. Zudem ist der Schlauchmantelabschnitt 11 auf eine noch näher zu beschreibende Art und Weise fluiddicht mit dem Wandabschnitt 13 der Mantelinnenfläche 6 verbunden. Auf diese Weise ist ein Verschluss 9 zwischen dem Radialdurchlass 8 und dem zweiten Schlauchende 3 gebildet, der den Axialdurchlass 7 in Richtung des zweiten Schlauchendes 3 einseitig verschließt.

Wie anhand Fig. 3 ersichtlich ist, weist die Trennlinie 10 einen ersten axial verlaufenden Linienabschnitt 14, einen im Wesentlichen halbkreisförmigen zweiten Linienabschnitt 15 und einen dritten im Wesentlichen antiparallel zu dem ersten Linienabschnitt 14 beabstandet verlaufenden Linienabschnitt 16 auf. Derart ergibt sich ein zungenförmiger Verlauf 17 der Trennlinie 10.

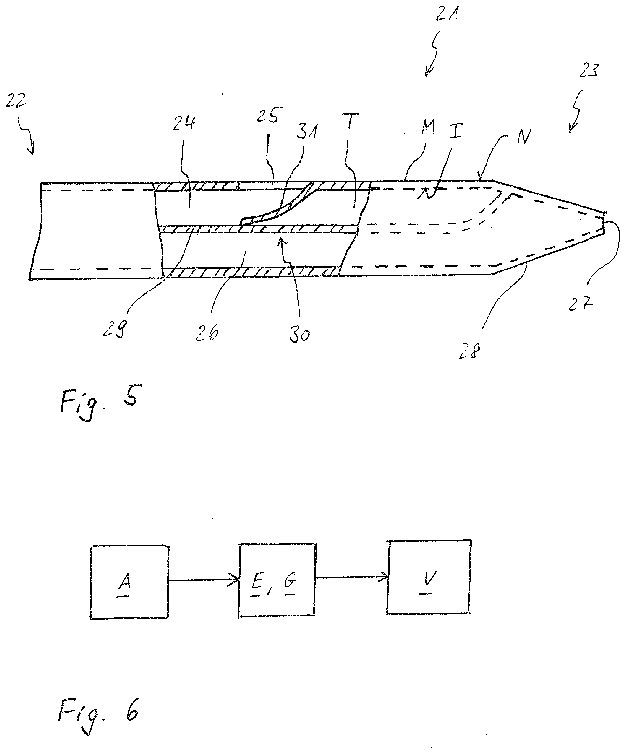

Eine zweite Ausführungsform der Erfindung nach der Fig. 5 ist für die Verwendung im Bereich der Medizintechnik, insbesondere für die Verwendung bei der Infusionstherapie, vorgesehen. Diese Ausführungsform weist als Schlauchleitung einen medizinischen Katheterschlauch 21 auf, wobei das erste Schlauchende ein proximales Katheterschlauchende 22, das zweite Schlauchende ein distales Katheterschlauchende 23, der Axialdurchlass ein erstes Lumen 24 und der Radialdurchlass ein Katheternebenkanal 25 ist. Der Katheterschlauch 21 weist zudem ein zweites Lumen 26, das zwischen dem proximalen Katheterschlauchende 22 und dem distalen Katheterschlauchende 23 erstreckt ist und in eine Austrittsöffnung 27 einer Katheterspitze 28 mündet. Das erste Lumen 24 und das zweite Lumen 26 sind durch eine im Wesentlichen axial erstreckte Trennwand 29 fluiddicht voneinander getrennt. Zum fluiddichten Verschließen eines Totraumvolumens T zwischen der Katheterspitze 28 und dem Katheternebenkanal 25 ist ein Verschluss 30 in Gestalt eines im Wesentlichen radial nach innen gewölbten Schlauchmantelabschnitts 31 des Katheterschlauchs 21 vorgesehen. Der Schlauchmantelabschnitt 31 ist entlang einer nicht näher bezeichneten und im Übrigen in analoger Weise zu der Trennlinie 10 des ersten Ausführungsbeispiels nach den Fig. 3 und 4 auf der Mantelaußenfläche N verlaufenden Trennlinie abschnittsweise von dem Mantel M des Katheterschlauchs 21 getrennt. Zum fluiddichten Verschließen des Totraumvolumens T ist der Schlauchmantelabschnitt 31 fluiddicht mit der Trennwand 29 und der angrenzenden Mantelinnenfläche I des Katheterschlauchs 21, genauer: des ersten Lumens 24, fluiddicht verbunden.

Wie anhand Fig. 6 ersichtlich ist, wird bei dem erfindungsgemäßen Verfahren zur Herstellung einer Schlauchleitung nach den vorgehenden Ausführungen zunächst der Schlauchmantel 4, 21 entlang einer Trennlinie aufgetrennt (A), der derart erhaltene Schlauchmantelabschnitt 11, 31 wird in radialer Richtung eingewölbt (E) und an der Mantelinnenfläche angelegt (G) und sodann fluiddicht mit dieser verbunden (V). Bei einer nicht näher schematisch dargestellten Ausführungsform des erfindungsgemäßen Verfahrens erfolgt das Auftrennen A mittels Laserschneidens und das fluiddichte Verbindung V mittels Laserschweißens

## Patentansprüche

1. Schlauchleitung (1, 21) mit
- einem zwischen einem ersten Schlauchende (2, 22) und einem zweiten Schlauchende (3, 23) axial erstreckten Schlauchmantel (4, M) mit einer Mantelaußenfläche (5, N) und einer Mantelinnenfläche (6, I),
- mindestens einem von der Mantelinnenfläche (6, I) umschlossenen und zwischen den Schlauchenden (2, 22; 3, 23) axial erstreckten Axialdurchlass (7, 24),
- mindestens einem im Wesentlichen radial durch den Schlauchmantel (4, M) erstreckten Radialdurchlass (8, 25), der den mindestens einen Axialdurchlass (7, 24) fluidleitend mit der Mantelaußenfläche (5, N) verbindet und
- mindestens einem zwischen dem Radialdurchlass (8, 25) und dem zweiten Schlauchende (3, 23) angeordneten und den mindestens einen Axialdurchlass (7, 24) in Richtung des zweiten Schlauchendes (3, 23) fluiddicht verschließenden Verschluss (9, 30),
**dadurch gekennzeichnet, dass**
- dass der mindestens eine Radialdurchlass (8, 25) durch einen im Wesentlichen radial nach innen gewölbten Schlauchmantelabschnitt (11, 31) gebildet ist, der abschnittsweise von dem übrigen Schlauchmantel (4, M) entlang einer Trennlinie (10) getrennt ist, wobei
- der mindestens eine Verschluss (9, 30) dadurch gebildet ist, dass der Schlauchmantelabschnitt (11, 31) an der Mantelinnenfläche (6, I) angelegt und fluiddicht mit dieser verbunden ist.

2. Schlauchleitung (1, 21) nach Anspruch 1, **dadurch gekennzeichnet, dass** die Trennlinie (10) in einer radial auf den Radialdurchlass (8, 25) gerichteter Blickrichtung einen zungenförmigen Verlauf (17) aufweist.

3. Schlauchleitung (1, 21) nach Anspruch 1 oder 2, **dadurch gekennzeichnet, dass** die Schlauchleitung ein medizinscher Katheterschlauch (21), insbesondere ein Zentralvenenkatheter, das erste Schlauchende ein proximales Katheterschlauchende (22), das zweite Schlauchende ein distales Katheterschlauchende (23), der Axialdurchlass ein erstes Lumen (24) und der Radialdurchlass ein Katheternebenkanal (25) ist.

4. Schlauchleitung (1, 21) nach Anspruch 3, **dadurch gekennzeichnet, dass** zumindest ein zweites Lumen (26) vorgesehen ist, das durch eine axial erstreckte Trennwand (29) fluiddicht von dem ersten Lumen (24) getrennt ist, wobei der Schlauchmantelabschnitt (31) fluiddicht mit der Trennwand (29) verbunden ist.

5. Verfahren zur Herstellung einer Schlauchleitung (1, 21) nach einem der vorhergehenden Ansprüche aufweisend die Schritte:
- Auftrennen (A) des Schlauchmantels (4, M) entlang der Trennlinie (10);
- Einwölben (E) des derart erhaltenen Schlauchmantelabschnitts (11, 31) in radialer Richtung und Anlegen (G) des Schlauchmantelabschnitts (11, 31) an der Mantelinnenfläche (6, I);
- fluiddichtes Verbinden (V) des Schlauchmantelabschnitts (11, 31) mit der Mantelinnenfläche (6, I).

6. Verfahren nach Anspruch 5, wobei das Auftrennen (A) mittels Laserschneidens erfolgt.

7. Verfahren nach einem der Ansprüche 5 bis 7, wobei das fluiddichte Verbinden (V) mittels Laserschweißens erfolgt.

## Claims

1. Hose line (1, 21) with
- a hose sheath (4, M) which extends axially between a first hose end (2, 22) and a second hose end (3, 23) and has a sheath outer face (5, N) and a sheath inner face (6, I),
- at least one axial passage (7, 24) enclosed by the sheath inner face (6, I) and extending axially between the hose ends (2, 22; 3, 23),
- at least one radial passage (8, 25) which extends substantially radially through the hose sheath (4, M) and fluidically connects the at least one axial passage (7, 24) to the sheath outer face (5, N), and
- at least one closure (9, 30) which is arranged between the radial passage (8, 25) and the second hose end (3, 23) and closes the at least one axial passage (7, 24) in a fluid-tight manner in the direction of the second hose end (3, 23),
**characterized in that**
- the at least one radial passage (8, 25) is formed by a hose sheath portion (11, 31) which is bent substantially radially inwards and which is partially separated from the rest of the hose sheath (4, M) along a partition line (10), wherein
- the at least one closure (9, 30) is formed by means of the hose sheath portion (11, 31) being placed onto the sheath inner face (6, I) and being connected thereto in a fluid-tight manner.

2. Hose line (1, 21) according to Claim 1, **characterized in that** the partition line (10) has a tongue-shaped profile (17) in a viewing direction directed radially onto the radial passage (8, 25).

3. Hose line (1, 21) according to Claim 1 or 2, **characterized in that** the hose line is a medical catheter hose (21), in particular a central venous catheter, the first hose end is a proximal catheter hose end (22), the second hose end is a distal catheter hose end (23), the axial passage is a first lumen (24), and the radial passage is a catheter side channel (25).

4. Hose line (1, 21) according to Claim 3, **characterized in that** at least one second lumen (26) is provided which is separated from the first lumen (24) in a fluid-tight manner by an axially extending partition wall (29), wherein the hose sheath portion (31) is connected in a fluid-tight manner to the partition wall (29).

5. Method for producing a hose line (1, 21) according to one of the preceding claims, said method comprising the steps:
- opening-up (A) the hose sheath (4, M) along the partition line (10);
- bending inwards (E) the thus obtained hose sheath portion (11, 31) in the radial direction, and placing (G) of the hose sheath portion (11, 31) onto the sheath inner face (6, I);
- connecting (V) the hose sheath portion (11, 31) in a fluid-tight manner to the sheath inner face (6, I).

6. Method according to Claim 5, wherein the opening-up (A) is effected by means of laser cutting.

7. Method according to any one of Claims 5 to 7, wherein the fluid-tight connection (V) is effected by means of laser welding.

## Revendications

1. Conduite en tuyau souple (1, 21) comprenant
- une gaine de tuyau (4, M) qui s'étend axialement entre une première extrémité de tuyau (2, 22) et une deuxième extrémité de tuyau (3, 23) et qui comporte une surface de gaine extérieure (5, N) et une surface de gaine intérieure (6, I),
- au moins un passage axial (7, 24) qui est entouré par la surface de gaine intérieure (6, I) et qui s'étend axialement entre les extrémités de tuyau (2, 22 ; 3, 23),
- au moins un passage radial (8, 25) qui s'étend sensiblement radialement à travers la gaine de tuyau (4, M) et qui relie fluidiquement l'au moins un passage axial (7, 24) à la surface de gaine extérieure (5, N) et
- au moins une fermeture (9, 30) qui est disposée entre le passage radial (8, 25) et la deuxième extrémité de tuyau (3, 23) et qui ferme de manière étanche aux fluides l'au moins un passage axial (7, 24) en direction de la deuxième extrémité de tuyau (3, 23), **caractérisée en ce que**
- l'au moins un passage radial (8, 25) est formé par une portion de gaine de tuyau (11, 31) incurvée sensiblement radialement vers l'intérieur et qui est partiellement séparée de la gaine de tuyau restante (4, M) le long d'une ligne de séparation (10),
- l'au moins une fermeture (9, 30) est formée par le fait que la portion de gaine de tuyau (11, 31) est appliquée sur la surface de gaine intérieure (6, I) et est reliée à celle-ci de manière étanche aux fluides.

2. Conduite en tuyau souple (1, 21) selon la revendication 1, **caractérisée en ce que** la ligne de séparation (10) a une allure en forme de languette (17) lorsque l'on regarde dans une direction orientée radialement vers le passage radial (8, 25).

3. Conduite en tuyau souple (1, 21) selon la revendication 1 ou 2, **caractérisée en ce que** la conduite en tuyau souple est un tuyau de cathéter médical (21), en particulier un cathéter veineux central, la première extrémité de tuyau est une extrémité de tuyau de cathéter proximale (22), la deuxième extrémité de tuyau est une extrémité de tuyau de cathéter distale (23), le passage axial est une première lumière (24) et le passage radial est un conduit secondaire de cathéter (25).

4. Conduite en tuyau souple (1, 21) selon la revendication 3, **caractérisée en ce qu'**au moins une deuxième lumière (26) est prévue qui est séparée de manière étanche aux fluides de la première lumière (24) par une paroi de séparation (29) s'étendant axialement, la portion de gaine de tuyau (31) étant reliée de manière étanche aux fluides à la paroi de séparation (29).

5. Procédé de fabrication d'une conduite en tuyau souple (1, 21) selon l'une des revendications précédentes, comprenant les étapes suivantes :
- séparer (A) la gaine de tuyau (4, M) le long de la ligne de séparation (10) ;
- courber (E) la portion de gaine de tuyau (11, 31) ainsi obtenue dans la direction radiale et appliquer (G) la portion de gaine de tuyau (11, 31) sur la surface de gaine intérieure (6, I) ;
- relier de manière étanche aux fluides (V) la portion de gaine de tuyau (11, 31) à la surface de gaine intérieure (6, I).

6. Procédé selon la revendication 5, la séparation (A) étant effectuée par découpe au laser.

7. Procédé selon l'une des revendications 5 à 7, la liaison étanche aux fluides (V) étant réalisée par soudage au laser.
